# EUROPEAN PATENT SPECIFICATION

(11) **EP 2 474 309 B1**
(45) Date of publication and mention of the grant of the patent: **29.08.2018**
(21) Application number: 10813374.5
(22) Date of filing: 22.03.2010
(51) Int. Cl.: A61K 31/192, A61K 31/137, A61K 31/198, A61P 29/00, A61P 25/04, A61K 47/10, A61K 9/08, A61K 9/16, A61K 9/20, A61K 9/28, A61K 47/18

(54) **PHARMACEUTICAL COMPOSITION COMPRISING IBUPROFEN, TRAMADOL AND A BASIC AMINO ACID, PREPARATION METHOD THEREOF AND USE OF SAME**
PHARMAZEUTISCHE ZUSAMMENSETZUNG MIT IBUPROFEN, TRAMADOL UND EINER BASISCHEN AMINOSÄURE, HERSTELLUNGSVERFAHREN DAFÜR UND VERWENDUNG DAVON
COMPOSITION PHARMACEUTIQUE À BASE D'IBUPROFÈNE, DE TRAMADOL ET D'UN AMINOACIDE BASIQUE, PROCEDÉ POUR SA PRÉPARATION ET UTILISATION DE CETTE DERNIÈRE

(30) Priority: 04.09.2009 ES 200901823
(43) Date of publication of application: 11.07.2012
(73) Proprietor: Farmalider, S.A., 28108 Alcobendas (Madrid) (ES)
(72) Inventor: JIMÉNEZ REDONDO, Ana, 28108 Alcobendas (Madrid) (ES); SANZ MENÉNDEZ, Nuria, 28108 Alcobendas (Madrid) (ES); GÓMEZ CALVO, Antonia, 28108 Alcobendas (Madrid) (ES); MARTÍNEZ-ALZAMORA, Fernando, 28108 Alcobendas (Madrid) (ES); MUÑOZ RUIZ, Ángel, 28108 Alcobendas (Madrid) (ES); HERNÁNDEZ HERRERO, Gonzalo, 28108 Alcobendas (Madrid) (ES)
(74) Representative: Rodriguez Oca, Jesus
(86) International application number: PCT/ES2010/000114
(87) International publication number: WO 2011/027009

(56) References cited:
- EP-A1- 1 964 552
- WO-A1-2004/026291
- WO-A1-2008/115572
- WO-A1-2008/150324
- US-A- 5 164 398
- RICO, M.A. ET AL.: 'Evaluacion de tramadol como un opioide alternativo a la codeina en el segundo peldano de la escala analgesica de la OMS' REV. SOC. ESP. DOLOR vol. 7, no. 6, August 2000 - September 2000, pages 345 - 353, XP008154162

## Description

### Field of the invention

The present invention relates to a pharmaceutical composition of ibuprofen, tramadol and a basic amino acid for administration by oral route, to a process for preparing it and to the use thereof, in particular, for the treatment of chronic or acute pain of moderate to severe intensity, inflammation, fever and/or other ailments wherein ibuprofen and/or tramadol are traditionally used.

### State of the art

The International Common Denomination, ICD, of ibuprofen is (±)-2-(4-isobutylphenyl)propionic acid (Merck Index, 12^{th} Edition, No. 4925). Ibuprofen is a non-steroidal anti-inflammatory drug (of those commonly called NSAIs) with analgesic, antipyretic and anti-inflammatory activity.

For years, pharmaceutical compositions of ibuprofen have been commercialised which, in general, contain ibuprofen in its racemic form. It is well-known that the active enantiomer of ibuprofen is the (S) form, although it has been demonstrated that mammals have an isomerase capable of converting the (R) form into the active (S) form.

Arginine and lysine salts of ibuprofen have been known since 1976. For example, Spanish patent ES 435416 discloses the preparation of lysine and arginine salts of ibuprofen, and indicates that these salts are moderately soluble.

Various specific compositions have also been disclosed which comprise a mixture of ibuprofen and any of the basic amino acids arginine or lysine. In general, in order to resolve stability and/or solubility problems, said compositions must meet certain strict conditions. Thus, United States patent US 4.834.966 discloses ibuprofen compositions in the form of granules for oral solution which are characterised by total solubility; however, for this to be the case, they must contain 33% - 46% of ibuprofen, 34% - 51% of L-arginine, 9% - 29% of sodium bicarbonate; the molar proportion between arginine and ibuprofen must range between 1.1 and 1.5, and the proportion by weight between sodium bicarbonate and ibuprofen must range between 0.25 and 0.75. Data are supplied which show that these compositions present a greater absorption and a faster onset of the analgesic effect. European patent application EP 0 710 108 A1 discloses liquid pharmaceutical compositions for oral administration that are physically and chemically stable through time, presenting excess arginine. In particular, said compositions are composed of arginine and ibuprofen, where the molar proportion between them must range between 1.1 and 1.5, and the concentration of ibuprofen is equal to or greater than 200 mg/ml, with optional excipients.

It is believed that ibuprofen, as most NSAIs, exerts its analgesic action through the inhibition of the cyclooxygenase enzyme (COX), the main agent responsible for the synthesis of prostaglandins. However, there are several variants of said enzyme. Cyclooxygenase-1 (COX-1) is a constitutive enzyme that protects the gastric mucous membrane under normal conditions and cyclooxygenase-2 (COX-2) is an inducible enzyme that is primarily involved in inflammation. Ibuprofen inhibits both cyclooxygenase-2 (COX-2) and cyclooxygenase-1 (COX-1). For this reason, the analgesic, antipyretic and anti-inflammatory action of ibuprofen is attributed to the inhibition of COX-2.

Although ibuprofen is safer than other NSAIs, it also causes gastric lesions and gastric bleeding. These adverse effects of ibuprofen on the gastrointestinal tract are attributed to the inhibition of COX-1.

Recently, it has been demonstrated that the combination ibuprofen-arginate causes less gastrointestinal adverse effects than ibuprofen alone (Dig. Dis. Sci. 2004 Sep; 49(9): 1538-44). A possible explanation lies in the discovery that the nitric oxide (NO) molecule may contribute to protect the gastric mucous membrane, and it is well-known that L-arginine is a substrate for NO-synthase and, consequently, the synthesis of NO increases.

The International Common Denomination (ICD) of tramadol is (±)-cis-2-[(dimethylamino)methyl]-1-(3-methoxyphenyl)cyclohexanol. This compound is specifically disclosed in United States patent No. 3.652.589. Tramadol is an analgesic that acts at the central level. It is used in the treatment of moderate to severe pain, of chronic or acute origin. It may also be used as a pre-operative analgesic, as a complement to surgical anaesthesia and in diagnostic exploration processes that evolve with pain.

The mechanism of action whereby tramadol exerts its analgesic effect is not completely opioid or completely non-opioid. It is a dual mechanism of action, since, on the one hand, it acts on the µ-opioid receptors, whereto it binds with a low affinity, and, on the other hand, it inhibits the recapture of noradrenaline and serotonin, which increases the concentration of these neurotransmitters in localised areas of the brain, thereby reducing the threshold of pain (J. Pharmacol. Exp. Ther. 1992 Jan; 260(1): 275-85). Thus, tramadol has been frequently described as an "atypical" opioid analgesic.

Tramadol has an action that is between 5 and 10 times less potent than that of morphine. It is well-known that typical opioids, such as morphine, cause characteristic undesireable secondary effects, for example, respiratory depression, constipation and sedation. Advantageously, said effects are much less marked and less common when tramadol is used. Although initially it was believed that it had a low potential for abuse, more recently some cases of adverse effects have been reported, including cases of abuse, in opioid-dependent patients (J. Anal. Toxicol. 1997 Nov-Dec; 21(7): 529-37). The most common adverse effects of tramadol are nausea, vomiting, perspiration and constipation.

Combinations of active principles with analgesic activity (such as opioids, NSAIs, etc.), which have a different mechanism of action and present synergic effects, are of interest, since they make it possible to achieve the desired analgesic effect using a lower quantity of active principles, thereby reducing the adverse effects.

The combined use of various analgesics, amongst them tramadol and ibuprofen, in odontology has been disclosed (Current. Therapeutic Research vol. 49, No. 3, March 1991, and Anesth. Prog. 53: 78-82, 2006). Compositions have also been disclosed which comprise combinations of analgesics, such as paracetamol with codeine or paracetamol with dextropropoxyphene.

Moreover, compositions that comprise combinations with tramadol have been disclosed. For example, international application WO 93/04675 A1 discloses a combination of tramadol and acetaminophen, acetaminophen being an analgesic and antipyretic agent that is not considered to be an NSAI due to its limited anti-inflammatory activity. European patent application EP 0 546 676 A1 discloses compositions which comprise a tramadol material, including its N-oxide derivative and its O-demethylated derivative and mixtures thereof, with a non-steroidal anti-inflammatory agent (NSAI), in particular ibuprofen. These two patent applications do not specifically disclose any pharmaceutical composition of the combination; they simply exemplify aqueous solutions with Tween® 80 and different proportions of tramadol and paracetamol or ibuprofen to be injected in mice.

International application WO 2008/092219 A2 discloses a combination of tramadol and ketoprofen. This application provides a single composition containing tramadol and ketoprofen, wherein both active principles are not isolated from one another, and it demonstrates that this is an unstable composition due to the interaction between both active principles (examples 8 and 9 of WO 2008/092219 A2). For this reason, said document proposes as a solution a composition that comprises the active principles isolated from one another or the administration thereof in separate pharmaceutical forms.

On the other hand, although, apparently, the combination of ibuprofen with arginine or lysine in certain compositions may entail beneficial effects with respect to the activity of ibuprofen, the literature does not disclose any specific compositions that comprise the combination tramadol and L-arginine. In fact, according to what has been described in the literature, combining them would be advised against, in light, for example, of the article by Ipek Yalcin et al. (Pharmacology Biochemistry and Behavior 80 (2005), 69-75), who concluded that their results indicated that L-arginine reduces the analgesic effect of tramadol.

For all these reasons, there is still a need to make available improved analgesic compositions with respect to those described in the state of the art, for example, with at least one of the following advantages: an adequate or improved solubility and stability, which make it possible to reduce the doses of the active ingredients in order to achieve the same analgesic activity, with less adverse effects and a faster onset of the action thereof.

### Object of the invention

Despite what has been disclosed in the literature, the present inventors have found that oral compositions which comprise a combination of ibuprofen, tramadol and a basic amino acid selected from arginine and lysine are advantageous as compared to those disclosed in the state of the art, presenting at least one of the advantages mentioned in the preceding paragraph.

Thus, a first aspect of the invention relates to a pharmaceutical composition for administration by oral route which comprises tramadol, or one of the pharmaceutically acceptable salts thereof, ibuprofen and a basic amino acid selected from arginine, lysine and the mixtures thereof, a composition wherein the molar proportion between the amino acid and ibuprofen ranges between 0.9:1 and 1.1:1.

A second aspect of the invention relates to a process for preparing said composition, a process which comprises mixing tramadol, or one of the pharmaceutically acceptable salts thereof, ibuprofen and the aforementioned basic amino acid with at least one pharmaceutically acceptable excipient.

According to a third aspect, the invention relates to the use of said composition for the manufacturing of a medicament designed for the treatment of at least one ailment selected from pain, inflammation, fever or any other ailment wherein ibuprofen and/or tramadol are used. Alternatively, this third aspect of the invention may also be formulated as a method for the treatment of at least one ailment selected from pain, inflammation, fever, or any other ailment that may be alleviated with ibuprofen and/or tramadol, a method which comprises administering a composition according to the invention to a mammal, preferably a human being, that needs it.

The inclusion of the amino acid, in particular arginine, in the compositions of the invention provides a gastroprotective effect. Thus, advantageously, the compositions of the invention make it possible to obtain the beneficial effects of the analgesic combination of tramadol and ibuprofen with less gastrointestinal adverse effects.

### Detailed description of the invention

### Tramadol

Tramadol and the pharmaceutically acceptable acid addition salts thereof are understood to mean the compound (±)-cis-2-[(dimethylamino)methyl]-1-(3-methoxyphenyl)cyclohexanol and the pharmaceutically acceptable acid addition salts thereof, including its solvates, polymorphs, stereoisomers and mixtures of stereoisomers, in particular its racemates.

The pharmaceutically acceptable acid addition salts may be prepared by conventional methods that are well-known to persons skilled in the art, using pharmaceutically acceptable, substantially non-toxic organic or inorganic acids. Such acids include hydrochloric, nitric, sulfuric, phosphoric, acetic, propionic, maleic, malonic, succinic, citric, tartaric, malic, salicylic, phthalic acids, etc. Preferably, hydrochloric acid is used.

Preferably, in the compositions of the invention, tramadol is found in the form of the hydrochloride salt thereof. It is especially preferred that the tramadol be in the form of tramadol hydrochloride in racemic form.

Tramadol is commercially available from several sources and may also be prepared following the process disclosed in United States patent No. 3.652.589.

### Ibuprofen

The term ibuprofen, as understood herein, includes ibuprofen in its racemic form ((R,S)-ibuprofen), the (S) enantiomer of ibuprofen ((S)-ibuprofen) and a mixture of the (R) and (S) enantiomers of ibuprofen, preferably enriched in the (S) form. Preferably, the ibuprofen used in the compositions of the invention is selected from (R,S)-ibuprofen and (S)-ibuprofen. It is especially preferred that the compositions of the invention contain (R,S)-ibuprofen.

Ibuprofen is commercially available from several sources and may also be prepared following the process disclosed in patent application GB 971700 A. The resolution of ibuprofen into its enantiomers is described in the article by Brushan et al., Biomed. Chromatogr., 1998, 12, 309.

In general, the compositions of the invention are prepared from ibuprofen and the basic amino acid in its free form, i.e. not in the form of a salt. In some compositions, in particular liquid compositions, the ibuprofen salt is usually obtained with the basic amino acid during the manufacturing process. In an alternative embodiment of the invention, the compositions are prepared from the ibuprofen salt with the basic amino acid. Said salts may be prepared, for example, as described in Spanish patent application ES 435416. Preferably, the solid compositions of the invention are prepared from ibuprofen and the basic amino acid in its free form and are maintained in free form in the final solid compositions.

### Amino acids

The basic amino acids arginine and lysine may exist in the D-form and the L-form. Herein, arginine is understood to mean L-arginine, D-arginine and the mixtures thereof, and lysine is understood to mean L-lysine, D-lysine and the mixtures thereof.

Preferably, the basic amino acid is arginine; in particular, the basic amino acid is L-arginine.

### Quantity of active principles

Preferably, the molar proportion between the amino acid and ibuprofen ranges between 0.9:1 and 1.1:1. It is especially preferred that the molar proportion between the amino acid and the ibuprofen present in the compositions of the invention be 1:1.

Preferably, the proportion by weight between ibuprofen and tramadol ranges between 1:10 and 40:1; it is especially preferred that it range between 1:1 and 10:1.

Preferably, the compositions of the invention contain a dose of tramadol ranging between 20 and 300 mg. It is especially preferred that the dose of tramadol be selected from 25, 50, 75, 100, 150, 200 and 300 mg of tramadol hydrochloride. It is particularly preferred that the compositions of the invention use a dose of 75 mg of tramadol hydrochloride, in the form of granules, for oral solution and as tablets. It is totally preferred that, in the compositions of the invention in the form of oral solution, a dose of 37.5 mg of tramadol hydrochloride per ml of solution be used.

Preferably, the compositions of the invention contain a dose of ibuprofen selected from between 50 and 800 mg. It is especially preferred that the dose of ibuprofen be selected from 50, 100, 150, 200, 300, 400, 500, 600, 700 and 800 mg of (R,S)-ibuprofen. It is particularly preferred that the compositions of the invention in the form of granules for oral solution and in the form of tablets contain 400 mg of (R,S)-ibuprofen. It is totally preferred that the compositions of the invention in the form of oral solution contain 200 mg of (R,S)-ibuprofen per ml of solution.

According to a first preferred embodiment, the composition of the invention is a composition in solid form and contains the following quantities of ibuprofen and tramadol:
- between 50 and 800 mg of (R,S)-ibuprofen, or a therapeutically equivalent quantity of (S)-ibuprofen, in particular between 50 and 600 mg, and
- between 20 and 300 mg of tramadol, or a therapeutically equivalent quantity of one of the pharmaceutically acceptable salts thereof, in particular between 20 and 100 mg.

In a second preferred embodiment, the composition of the invention is a composition in liquid form and contains the following quantities of ibuprofen and tramadol:
- between 20 mg/ml and 200 mg/ml of (R,S)-ibuprofen, or a therapeutically equivalent quantity of (S)-ibuprofen, in particular between 100 and 200 mg/ml, and
- between 20 mg/ml and 200 mg/ml of tramadol, or a therapeutically equivalent quantity of one of the pharmaceutically acceptable salts thereof, in particular between 20 and 100 mg/ml.

A composition that comprises the following as the only active principles is particularly preferred:
- 400 mg of (R,S)-ibuprofen, or a therapeutically equivalent quantity of (S)-ibuprofen,
- 340 mg of arginine, and
- 75 mg of tramadol hydrochloride,
contained in a unit dose in the case of solid compositions, or in a volume of liquid selected from between 2 and 6 ml, especially for every 2 ml, in the case of liquid compositions.

Preferably, the liquid compositions according to the invention are oral solutions which comprise, for every ml of solution, the following as the only active principles:
- 200 mg of (R,S)-ibuprofen, or a therapeutically equivalent quantity of (S)-ibuprofen,
- 170 mg of arginine, and
- 37.5 mg of tramadol hydrochloride.

### Excipients

The compositions of the invention are designed to be administered by oral route. They may be formulated both as solid pharmaceutical forms (including tablets, capsules, granules, powders, pills, etc.) and liquid pharmaceutical forms (including solutions, syrups, suspensions, etc.). Preferably, the compositions of the invention are formulated in the form of powder or granules for oral solution, as oral solutions and as tablets. The tablets optionally include film-coated tablets, pills, effervescent tablets and dispersible tablets, optionally coated.

The compositions of the invention comprise at least one pharmacologically acceptable excipient. Different excipients may be used, depending on the pharmaceutical form. Thus, the following may be used, for example, for solid forms: diluting, disaggregating, binding, lubricating, wetting, anti-caking, sweetening, flavouring, appetising agents, etc. For liquid forms, the following vehicles may be used: water, glycols, alcohols, oils, etc., and preservative, flavouring, appetising, colouring, sweetening agents, etc.

In particular, when the pharmaceutical composition is in the form of a powder for oral solution, it may contain anti-caking agents such as colloidal anhydrous silica, tribasic calcium phosphate, magnesium trisilicate, talc and mixtures thereof; lubricant agents such as magnesium stearate, calcium stearate, glyceryl palmitostearate, magnesium oxide, sodium benzoate, sodium lauryl sulfate, sodium stearyl fumarate, stearic acid, talc, glyceryl behenate and mixtures thereof; wetting agents such as sodium lauryl sulfate, sorbitol, docusate sodium, poloxamer, polyoxyethylene stearate, sorbitan esters and mixtures thereof; sweetening agents such as aspartame, mannitol, sorbitol, sodium saccharin, sodium cyclamate, sucrose, dextrose, fructose, glucose, inulin, isomaltose, lactitol, maltose, maltol, mannitol, sucralose, trehalose, xylitol, thaumatin and mixtures thereof; flavouring and appetising agents, for example, mint aroma, menthol aroma, caramel aroma and lemon aroma, although any other adequate aroma may be present; moreover, said aromas may be commercially found in the form of mixtures with other substances in order to facilitate the dosing thereof; for example, menthol aroma may contain natural flavouring substances and gum arabic, and mint aroma may contain natural flavouring preparations, natural flavouring substances, flavouring substances identical to natural ones, maltodextrin, modified corn starch, glycerol triacetate and pulegone, and/or mixtures thereof.

The compositions in the form of granules for oral solution according to the invention may contain the same pharmaceutically acceptable excipients mentioned for the powder for oral suspension, but additionally using a liquid vehicle for the binding of the solid components, for example, purified water, meglumine or organic alcohols such as ethanol; and binding agents such as magnesium trisilicate, cellulose, starch, talc, hypromellose, polyvinylpyrrolidone, tribasic calcium phosphate and mixtures thereof.

Preferably, the compositions of the invention in the form of powder or granules for oral solution further comprise a bicarbonate of an alkali metal, especially sodium bicarbonate with an alkalinising function; as well as a surfactant, especially sodium lauryl sulfate with a wetting function.

The compositions according to the invention in the form of oral solution may contain preservative agents, for example, domiphen bromide, butylparaben, propylparaben, ethylparaben, methylparaben, benzyl alcohol, sodium acetate, glycerine, xylitol or mixtures thereof; appetising agents such as sodium chloride, ethylmaltol, ethyl vanilla, vanilla, menthol and mixtures thereof; flavouring agents, for example, mint aroma, menthol aroma, caramel aroma and lemon aroma, although any other adequate aroma may also be present; moreover, said aromas may be commercially found in the form of mixtures with other substances in order to facilitate the dosing thereof; thus, for example, caramel aroma may contain flavouring substances identical to natural ones, natural flavouring substances, maltodextrin, sugar, vegetable oil, silicon dioxide and lecithin, and mint aroma may contain natural flavouring preparations, natural flavouring substances, flavouring substances identical to natural ones, maltodextrin, modified corn starch, glycerol triacetate and pulegone; sweetening agents, for example, maltitol, sucrose, dextrose, fructose, glucose, glycerine, inulin, isomaltose, lactitol, maltose, maltol, mannitol, sucralose, trehalose, xylitol, propylene glycol, sorbitol, sodium saccharin, thaumatin, sodium cyclamate and/or mixtures thereof; and a liquid vehicle for the solution such as purified water.

Preferably, in the compositions of the invention in the form of oral solution domiphen bromide is used as a preservative agent. Advantageously, this preservative agent is effective at the basic pH resulting from the final formula. Preferably, in said solutions, the primary sweetening agent used is maltitol, specifically liquid maltitol. Advantageously, maltitol, as compared to sucrose, provides a lower solids load to the formulation, thereby allowing for a greater concentration of active ingredients, in addition to presenting a lower cariogenic power. In a preferred embodiment of the invention, the oral solutions contain a sweetening system composed of a mixture of liquid maltitol, sodium saccharin and thaumatin. Advantageously, this combination provides a potent sweetening effect.

The compositions according to the invention in the form of tablets may contain diluting agents such as, for example, corn starch, microcrystalline or powdered cellulose, mannitol, isomaltose, lactose, magnesium carbonate, hydrogenated calcium phosphate dihydrate, dicalcium phosphate, dextrose, sucrose or mixtures thereof; disaggregating agents such as corn and/or potato starch, sodium carboxymethyl aminopectin, alginic acid and the salts and derivatives thereof, formaldehyde-gelatin, casein formaldehyde, gelatin, sodium croscarmellose, sodium starch glycolate, microcrystalline cellulose or powdered cellulose, low-substituted hydroxypropylcellulose and mixtures thereof; adsorbent agents such as microcrystalline or powdered cellulose, colloidal anhydrous silica, aluminium-magnesium silicate, amorphous silicas such as Sipernat® and mixtures thereof; lubricating agents and anti-caking agents, for example, colloidal anhydrous silica, amorphous silicas such as Sipernat®, talc, magnesium stearate, calcium stearate, stearic acid, polyethylene glycol, Precirol®, sodium benzoate, sodium lauryl sulfate or mixtures thereof; anti-caking agents such as low-substituted hydroxypropylcellulose, ethylcellulose and mixtures thereof; and colouring agents. Furthermore, when the tablet is manufactured by wet granulation, a liquid vehicle is used for the binding of the solid components, for example, purified water, meglumine or organic alcohols; as well as binding agents such as magnesium trisilicate, cellulose, starch, talc, hypromellose, polyvinylpyrrolidone, tribasic calcium phosphate and mixtures thereof.

The tablets according to the invention may be optionally coated. The coated tablets may be prepared by means of a tabletting process or the incorporation of film coatings.

Thus, in the tabletting process, the following may be used: sugar as a coating agent; insulating and impermeabilising agents, such as cellulose acetophthalate, polyvinyl phthalate, acrylic resins; plasticising agents such as alkylic esters of phthalic acid, esters of citric acid or castor oil; and colouring agents.

In order to incorporate a film coating, the following may be used: film-forming polymers such as hydroxypropylmethylcellulose, Eudragit® polymers, cellulose acetophthalate, hydroxypropylmethylcellulose phthalates, polyvinyl acetophthalate, alginic acid and the derivatives thereof, cellulose hydrogen phthalate, ethylcellulose; plasticising agents such as propylene glycol, glycerine, triacetin, polyethylene glycol, acetylated monoglycerides, phthalate esters, castor oil, sebacic acid esters, silicones or mixtures thereof; opacifying agents such as titanium dioxide; lubricating agents such as talc; colouring agents; and a liquid vehicle for the dispersion or solution of these solid components, for example, purified water, meglumine or organic alcohols. Already-prepared, commercially available mixtures of said components may also be used, including, for example, hypromellose, titanium dioxide, colouring agent, macrogol 400 and talc.

The compositions according to the invention in the form of effervescent tablets comprise a solid system for the generation of carbon dioxide, generally a carbonate and/or bicarbonate of an alkali metal, such as sodium or potassium bicarbonate, jointly with an organic acid or an acid salt thereof, which react quickly in the presence of water. Organic acids include: citric acid, tartaric acid, ascorbic acid, malic acid, fumaric acid and maleic acid. Moreover, other excipients may be included, such as diluting agents, for example, lactose, calcium phosphate, calcium sulfate, calcium carboxymethylcellulose, microcrystalline or powdered cellulose, cellulose acetate, dextrates, dextrins, dextrose, fructose, glycerol palmitostearate, kaolin, lactitol, magnesium carbonate, magnesium oxide, maltitol, maltodextrins, maltose, polymethacrylates, pre-gelatinised starch, sodium chloride, starch, sucrose; binding agents such as polyvinylpyrrolidone, magnesium trisilicate, cellulose, starch, talc, tribasic calcium phosphate; sweetening agents such as mannitol, sorbitol, sodium saccharin, sodium cyclamate, aspartame, sucrose, dextrose, fructose, glucose, inulin, isomaltose, lactitol, maltose, maltol, mannitol, sucralose, trehalose, xylitol, thaumatin; lubricating agents such as magnesium stearate, calcium stearate, glyceryl palmitostearate, magnesium oxide, sodium benzoate, sodium lauryl sulfate, sodium stearyl fumarate, stearic acid, talc, glyceryl behenate, polyethylene glycol; anti-foaming agents such as simethicone emulsion; binding vehicles such as purified water; flavouring and appetising agents, and/or mixtures thereof.

The compositions of the invention in the form of dispersible tablets, optionally coated, comprise a solid disaggregating system which, in contact with water, causes a quick disaggregation of the tablets, such as alginic acid, carboxymethylcellulose, low-substituted hydroxypropylcellulose, microcrystalline or powdered cellulose, colloidal anhydrous silica, sodium croscarmellose, crospovidone, aluminium-magnesium silicate, methylcellulose, povidone, sodium alginate, sodium starch glycolate, starch or pre-gelatinised starch, and/or mixtures thereof. Other excipients may be additionally included in the dispersible tablets, such as, for example, diluting agents such as lactose, calcium phosphate, calcium sulfate, calcium carboxymethylcellulose, microcrystalline or powdered cellulose, cellulose acetate, dextrates, dextrins, dextrose, fructose, glyceryl palmitostearate, kaolin, lactitol, magnesium carbonate, magnesium oxide, maltitol, maltodextrins, maltose, polymethacrylates, pre-gelatinised starch, sodium chloride, starch, sucrose; disaggregating agents; binding agents such as polyvinylpyrrolidone, magnesium trisilicate, cellulose, starch, talc, tribasic calcium phosphate; sweetening agents such as mannitol, sorbitol, sodium saccharin, sodium cyclamate, aspartame, sucrose, dextrose, fructose, glucose, inulin, isomaltose, lactitol, maltose, maltol, mannitol, sucralose, trehalose, xylitol, thaumatin; lubricating agents such as magnesium stearate, calcium stearate, glyceryl palmitostearate, magnesium oxide, sodium benzoate, sodium lauryl sulfate, sodium stearyl fumarate, stearic acid, talc, glyceryl behenate, polyethylene glycol; flavouring and appetising agents, and/or mixtures thereof. The dispersible tablets may be optionally coated with adequate excipients, such as film-forming polymers, for example, hydroxypropylmethylcellulose, Eudragit®, cellulose acetophthalate, hydroxypropylmethylcellulose phthalates, polyvinyl acetophthalate, alginic acid and the derivatives thereof, cellulose hydrogen phthalate, ethylcellulose; plasticising agents such as propylene glycol, glycerine, triacetin, polyethylene glycol, acetylated monoglycerides, phthalate esters, castor oil, sebacic acid esters, silicones; and colouring agents, and/or mixtures thereof; in order to perform said coating, solvents may be used, such as methanol, methylene chloride, isopropanol, acetone, purified water, ethyl acetate, isopropanol and ethanol.

The compositions of the invention may be prepared according to conventional granulation, compression and dilution techniques, such as those described, for example, in Remington, "The Science and Practice of Pharmacy", Mack Publishing Company, 20th Edition, 2000.

Thus, according to a second aspect of the invention, the latter relates to a process for preparing a composition according to the invention, which comprises mixing tramadol or one of the pharmaceutically acceptable salts thereof, ibuprofen and a basic amino acid with at least one pharmaceutically acceptable excipient.

In a preferred embodiment, the compositions in the form of granules for oral solution are prepared by means of a process that comprises the following steps:
a) preparing a solution of tramadol, or one of the pharmaceutically acceptable salts thereof, preferably tramadol hydrochloride, and a binding agent,
b) granulating the ibuprofen with the solution prepared in the preceding step,
c) drying the granules, and
d) mixing the dry granules with arginine.

In another preferred embodiment, the compositions in the form of oral solution are prepared by means of a process that comprises the following steps:
a) dissolving the tramadol, or one of the pharmaceutically acceptable salts thereof, preferably tramadol hydrochloride, in a sufficient quantity of water,
b) subsequently adding a basic amino acid selected from arginine and lysine until the complete dispersion thereof, and
c) subsequently adding the ibuprofen until the complete dissolution thereof.

In a preferred embodiment, the compositions in the form of tablets are prepared by means of a process that comprises the following steps:
a) mixing tramadol, or one of the pharmaceutically acceptable salts thereof, and ibuprofen,
b) granulating the mixture obtained in the preceding step with a solution of a binding agent,
c) drying the granules obtained,
d) mixing the dry granules with arginine, and
e) compressing.

Typically, in the three preferred processes, at least one pharmaceutically acceptable excipient is added in one or more of the different steps described. Preferably, said excipients are selected from those described above for each pharmaceutical form.

These three preferred processes are particularly advantageous, since they make it possible to obtain homogeneous compositions, which is not a trivial issue in the case of the compositions of the invention that comprise three active ingredients (tramadol, ibuprofen and an amino acid). Moreover, the two processes for preparing solid forms prevent the formation, in the pharmaceutical form, of amino acid salts with a moderate solubility, which would hinder the manufacturing process. Furthermore, the process for preparing liquid oral solutions favours the dissolution of the active ingredients, thereby preventing the use of high temperatures.

The compositions of the invention have an analgesic effect and are useful for the treatment of pain, in particular chronic or acute pain, of moderate to severe intensity. They may be used for the treatment of various types of pain caused by different ailments, such as cephaleas, odontalgias, otalgias, dysmenohrrea, migraines, muscular pain, articular pain, neuropathic pain, post-traumatic pain, post-surgical pain and oncological pain. They may also be used as pre-operative analgesics, as a complement to surgical anaesthesia and in diagnostic exploration processes that evolve with pain. Moreover, the compositions may be used as anti-inflammatory agents and, primarily due to the action of ibuprofen, as antipyretic agents; for these reasons, they are useful for the treatment of the inflammation and/or fever caused by different ailments, such as the flu, rheumatoid arthritis (including juvenile rheumatoid arthritis), osteoarthritis, ankylosing spondylitis, arthrosis and other acute or chronic rheumatoid processes, bursitis, synovitis, capsulitis or other types of inflammatory traumatic or sports injuries.

The compositions of the invention are particularly useful for the treatment of pain and inflammation. They may also be useful for the treatment of other ailments that may be alleviated with ibuprofen or tramadol. Such ailments include, for example, acne.

Herein, the term "treatment" is understood to mean both the treatment of already-established symptoms and prophylactic treatment. Preferably, they are used for the treatment of established symptoms.

Below, the invention is described in greater detail on the basis of several illustrative examples which in no way limit the scope of the invention.

### Examples

### Example 1. Preparation of granules for oral solution

A composition of granules for oral solution was prepared with the quantitative composition detailed in the following table:

| **Ingredient** | **mg/ml** | **Function** |
|---|---|---|
| Active ingredients | | |
| Ibuprofen 70 µm | 400.0 | Active ingredient |
| Tramadol HCl | 75.0 | Active ingredient |

| Excipients | | |
|---|---|---|
| Arginine | 340.0 | Solubiliser |
| Hypromellose 6 cP | 25.0 | Binder |
| Aspartame (E-951) | 20.0 | Sweetener |
| Sucrose | 460.0 | Sweetener/Diluent |
| Sodium bicarbonate | 32.0 | Alkalinising agent |
| Sodium saccharin | 7.0 | Sweetener |
| Sodium lauryl sulfate | 1.0 | Wetting agent |
| Menthol aroma | 20.0 | Flavouring agent |
| Mint aroma | 20.0 | Flavouring agent |
| Purified water | s.q. | Vehicle |
| Weight | 1,400.0 | |

where:
- the menthol aroma contains natural flavouring substances and 79 % gum arabic (E-414),
- the mint aroma comprises natural flavouring preparations, natural flavouring substances, flavouring substances identical to natural ones, maltodextrin, 7.88 % modified corn starch (E-1450), 0.06 % glycerol triacetate (E-1518), and 0.1405 % pulegone, and
- the purified water disappears during the manufacturing process.

The composition was prepared according to the following process:
- The Ibuprofen, 30 % by weight of the sucrose and the sodium lauryl sulfate were run through a 1.0 mm sieve.
- It was mixed with a solution of Tramadol·HCl and hypromellose 6 cP in a sufficient quantity of purified water.
- The aforementioned mixture was introduced into a drying system and dried to a humidity lower than 0.5 % at a product temperature not greater than 40 °C.
- Once dried, the granules were run through a 0.8 mm sieve.
- On the basis of the yield (by weight) obtained in the preceding granules, the necessary quantity of the rest of the raw materials was re-calculated, and they were run through a 0.8 mm sieve.
- The sieved dry granules were mixed with the rest of the raw materials of the formulation, mixing them until homogeneity was achieved.
- They were dosed in complex sachets formed by heat-sealable Stucco/Aluminium/Resin.

### Example 2. Preparation of an oral solution

An oral solution was prepared with the quantitative composition detailed in the following table:

| **Ingredient** | **mg/ml** | **Function** |
|---|---|---|
| Active ingredients | | |
| Ibuprofen 40 µm | 200.00 | Active ingredient |
| Tramadol HCl | 37.50 | Active ingredient |

| Excipients | | |
|---|---|---|
| Arginine | 170.00 | Solubiliser |
| Domiphen bromide | 0.10 | Preservative |
| Liquid maltitol | 100.00 | Sweetener |
| Sodium saccharin | 1.70 | Sweetener |
| Caramel aroma | 2.50 | Flavouring agent |
| Mint aroma | 16.00 | Flavouring agent |
| Thaumatin (E-957) | 0.05 | Sweetener |
| Purified water | s.q.f. | Vehicle/Diluent |

where:
- the caramel aroma contains flavouring substances identical to natural ones, natural flavouring substances, flavouring preparations, maltodextrin, sugar, vegetable oil, 0.88 % silicon dioxide (E-551) and 0.60 % lecithin (E-322), and
- the mint aroma comprises natural flavouring preparations, natural flavouring substances, flavouring substances identical to natural ones, maltodextrin, 7.88 % modified corn starch (E-1450), 0.06 % glycerol triacetate (E-1518) and 0.1405 % pulegone.
- s.q.f. indicates sufficient quantity for.

The composition was prepared according to the following process:
- The Tramadol·HCl was dissolved in a sufficient quantity of purified water.
- Whilst under stirring, the following were added, one by one, in this order: sodium saccharin, caramel aroma, mint aroma and thaumatin (E-957), not adding the next until complete dissolution of the previous one.
- The arginine was added under stirring and it was stirred until total dispersion.
- Once the arginine was dispersed, and still under stirring, the Ibuprofen was added and it was stirred until the total dissolution thereof.
- The liquid maltitol was slowly added and it was stirred until complete homogenisation.
- The domiphen bromide was dissolved separately, under stirring, in a sufficient quantity of purified water and said solution was added to the solution of the previous step, jointly with a quantity of purified water used to wash said secondary reactor.
- The complete disappearance of bubbles and foam from the solution in the main tank was awaited and, if necessary, it was aided by means of a vacuum.
- It was made up to volume with purified water and stirred until total homogeneity was achieved.
- It was dosed in topaz glass jars, incorporating a dosing valve.

### Example 3. Preparation of film-coated tablets

Film-coated tablets were prepared with the quantitative composition detailed in the following table:

| **Ingredient** | **mg/tablet** | **Function** |
|---|---|---|
| Active ingredients | | |
| Ibuprofen 70 µm | 400.00 | Active ingredient |
| Tramadol HCl | 75.00 | Active ingredient |

| Excipients | | |
|---|---|---|
| Arginine | 340.00 | Solubiliser |
| Hypromellose 6 cP | 7.00 | Binding agent |
| Sodium croscarmellose | 10.00 | Disaggregating agent |
| Lactose monohydrate | 30.00 | Diluent |
| Microcrystalline cellulose 101 | 31.50 | Diluent/Disaggregating agent |
| Pre-gelatinised corn starch without gluten | 20.00 | Disaggregating agent |
| Colloidal anhydrous silica | 7.50 | Anti-caking agent |
| Magnesium stearate of vegetable origin | 9.00 | Lubricant |
| Purified water | s.q. | Vehicle |
| Weight of the core | 935.00 | |

| Coating | | |
|---|---|---|
| Opadry® 02B22026 yellow | 25.00 | Coating with a 2.7 % weight increase Coating |
| Purified water | s.q. | vehicle |
| Weight of the coated tablet | 960.00 | |

where:
- the purified water disappears during the manufacturing process,
- Opadry® 02B22026 yellow contains 62.5 % of hypromellose 5 cP (equiv. to 6.25 mg/tablet), 23.55 % of titanium dioxide (E-171) (equiv. to 2.35 mg/tablet), 6.5 % of quinoline yellow (E-104) (equiv. to 0.65 mg/tablet), 6.25 % of macrogol 400 (equiv. to 0.65 mg/tablet), 1.0 % of talc (equiv. to 0.10 mg/tablet) and 0.2 % of orange yellow S (E-110) (equiv. to 0.02 mg/tablet).
- s.q. indicates sufficient quantity.

The composition was prepared according to the following process:
- The ibuprofen, the tramadol·HCl and 1/5 of the sodium croscarmellose were mixed until homogeneity was achieved.
- It was mixed with a solution of hypromellose 6 cP in a sufficient quantity of purified water added very slowly.
- It was sieved at 3.0 mm.
- The preceding mixture was introduced into a drying system and dried to a humidity of less than 0.4 % at a product temperature not greater than 35 °C.
- Once the granules were dry, they were sieved at 1.5 mm.
- On the basis of the yield (by weight) obtained in the preceding granules, the necessary quantity of the rest of the raw materials was re-calculated, and they were sieved at 1.5 mm, except for the magnesium stearate, which was sieved at 0.8 mm.
- The sieved dry granules were mixed with the arginine, the remaining sodium croscarmellose (4/5), the lactose monohydrate, the microcrystalline cellulose, the pre-gelatinised corn starch without gluten and the colloidal anhydrous silica, mixing until homogeneity was achieved.
- The magnesium stearate was added to the preceding mixture, mixing until homogeneity was achieved.
- The final mixture obtained was compressed in punches with an oblong, biconcave, grooved format at 935.0 mg per tablet.
- The day prior to the coating, the coating suspension was prepared in an approximate percentage of 10 % or 20 % greater than the theoretical quantities thereof, in order to compensate for the inherent losses that occur during the coating process, dispersing the Opadry® 02B22026 yellow in a quantity of purified water until a homogeneous suspension is obtained.
- The cores were placed in the coating system and, after pre-heating them at 40°C - 43°C, they were coated with the previous suspension until a final weight of the coated tablets of 960.0 mg was obtained (approximately a 2.7 % weight increase).
- It was blister-packed in white opaque PVC/PVDC - Aluminium.

## Claims

1. Pharmaceutical composition for administration by oral route, **characterised in that** it comprises tramadol or one of the pharmaceutically acceptable salts thereof, ibuprofen and the basic amino acid arginine, where the molar proportion between the amino acid and ibuprofen ranges between 0.9:1 and 1.1:1.

2. Composition according to claim 1, **characterised in that** the basic amino acid is L-arginine.

3. Composition according to claims 1 or 2, **characterised in that** the ibuprofen is selected from the group formed by (R,S)-ibuprofen and (S)-ibuprofen.

4. Composition according to claim 3, **characterised in that** it comprises tramadol in the form of tramadol hydrochloride.

5. Composition according to any of the preceding claims, **characterised in that** it comprises a molar proportion between arginine and ibuprofen of 1:1.

6. Composition according to any of the preceding claims, in solid form, which contains the following quantities of ibuprofen and tramadol:
- between 50 and 800 mg of (R,S)-ibuprofen, or a therapeutically equivalent quantity of (S)-ibuprofen, and
- between 20 and 300 mg of tramadol, or a therapeutically equivalent quantity of one of the pharmaceutically acceptable salts thereof.

7. Composition according to any of the preceding claims, in liquid form, which contains the following quantities of ibuprofen and tramadol:
- between 20 mg/ml and 200 mg/ml of (R,S)-ibuprofen, or a therapeutically equivalent quantity of (S)-ibuprofen, and
- between 20 mg/ml and 200 mg/ml of tramadol, or a therapeutically equivalent quantity of one of the pharmaceutically acceptable salts thereof.

8. Composition according to any of claims 5 and 6, which comprises the following as the only active principles:
- 400 mg of (R,S)-ibuprofen, or a therapeutically equivalent quantity of (S)-ibuprofen,
- 340 mg of arginine, and
- 75 mg of tramadol hydrochloride
a) in a unit dose, if it is a solid composition, or
b) per every 2 to 6 ml, if it is a liquid composition.

9. Composition according to any of claims 1 to 8, the pharmaceutical form whereof is selected from the group formed by granules for oral solution, oral solutions and tablets.

10. Process for preparing a composition according to any of claims 1 to 8, which comprises mixing tramadol or one of the pharmaceutically acceptable salts thereof, ibuprofen and said basic amino acid with at least one pharmaceutically acceptable excipient.

11. Process according to claim 10, where said composition is found in the form of granules for oral solution, **characterised in that** it comprises the following steps:
a) granulating the ibuprofen with a solution of tramadol, or one of the pharmaceutically acceptable salts thereof, and a binding agent,
b) drying the granules,
c) mixing the dry granules with arginine.

12. Process according to claim 10, where said composition is found in the form of an oral solution, **characterised in that** it comprises the following steps:
a) dissolving the tramadol, or one of the pharmaceutically acceptable salts thereof, in a sufficient quantity of water,
b) subsequently adding the basic amino acid until total dispersion, and
c) subsequently adding the ibuprofen until the total dissolution thereof.

13. Process according to claim 10, where said composition is found in the form of a tablet, **characterised in that** it comprises the following steps:
a) mixing the tramadol, or one of the pharmaceutically acceptable salts thereof, and ibuprofen,
b) granulating the mixture obtained in the preceding step with a solution of a binding agent,
c) drying the granules obtained,
d) mixing the dry granules with arginine,
e) compressing.

14. Use of a composition according to any of claims 1 to 9, for the manufacturing of a medicament designed for the treatment of at least one ailment selected from pain, inflammation and fever, or any other ailment that may be alleviated with ibuprofen and/or tramadol.

15. Use according to claim 14, **characterised in that** said pain is a chronic or acute pain, of moderate to severe intensity.

16. Use according to any of claims 14 or 15, **characterised in that** the pain is selected from the group formed by cephalea, odontalgia, otalgia, dysmenorrhea, migraine, muscular pain, articular pain, osteoarthritis, neuropathic pain, post-traumatic pain, post-surgical pain and oncological pain.

## Patentansprüche

1. Pharmazeutische Zusammensetzung zur oralen Verabreichung, **dadurch gekennzeichnet, dass** sie Tramadol oder eines seiner pharmazeutisch verträglichen Salze, Ibuprofen und die basische Aminosäure Arginin umfasst, wobei das molare Verhältnis zwischen der Aminosäure und Ibuprofen zwischen 0.9:1 und 1.1: 1 liegt.

2. Zusammensetzung gemäss Anspruch 1, **dadurch gekennzeichnet, dass** die basische Aminosäure L-Arginin ist.

3. Zusammensetzung gemäss Ansprüche 1 oder 2, **dadurch gekennzeichnet , dass** das Ibuprofen aus der Gruppe bestehend aus (R, S) -Ibuprofen und (S) -Ibuprofen ausgewählt wird.

4. Zusammensetzung gemäss Anspruch 3, **dadurch gekennzeichnet, dass** sie Tramadol in Form von Tramadolhydrochlorid enthält.

5. Zusammensetzung gemäss einem der vorhergehenden Ansprüche, **dadurch gekennzeichnet, dass** sie ein molares Verhältnis zwischen Arginin und Ibuprofen von 1: 1 aufweist.

6. Zusammensetzung gemäss einem der vorhergehenden Ansprüche in fester Form, die die folgenden Mengen an Ibuprofen und Tramadol enthält:
- Zwischen 50 und 800 mg (R,S)-Ibuprofen, oder eine therapeutisch äquivalente Menge an (S)-Ibuprofen, und
- Zwischen 20 und 300 mg Tramadol, oder eine therapeutisch äquivalente Menge eines seiner pharmazeutisch verträglichen Salze.

7. Zusammensetzung gemäss einem der vorhergehenden Ansprüche in flüssiger Form, die die folgenden Mengen an Ibuprofen und Tramadol enthält:
- Zwischen 20 mg/ml und 200 mg/ml (R,S)-Ibuprofen, oder eine therapeutisch äquivalente Menge an (S)-Ibuprofen, und
- Zwischen 20 mg/ml und 200 mg/ml Tramadol, oder eine therapeutisch äquivalente Menge eines seiner pharmazeutisch verträglichen Salze.

8. Zusammensetzung nach einem der Ansprüche 5 und 6, die als einziges aktives Prinzip folgendes umfaßt:
- 400 mg (R,S)-Ibuprofen, oder eine therapeutisch äquivalente Menge an (S)-Ibuprofen,
- 340 mg Arginin, und
- 75 mg Tramadolhydrochlorid
a) in einer Einheitsdosis, wenn es sich um eine feste Zusammensetzung handelt
b) pro 2 bis 6 ml, wenn es sich um eine flüssige Zusammensetzung handelt.

9. Zusammensetzung nach einem der Ansprüche 1 bis 8, deren pharmazeutische Form ausgewählt wird aus der Gruppe, die aus Granulaten für die orale Lösung, oralen Lösungen und Tabletten besteht.

10. Verfahren zur Herstellung einer Zusammensetzung gemäss einem der Ansprüche 1 bis 8, umfassend das Mischen von Tramadol oder eines seiner pharmazeutisch verträglichen Salze, Ibuprofen und besagte basische Aminosäure mit mindestens einem pharmazeutisch verträglichen Hilfsstoff.

11. Verfahren nach Anspruch 10, wobei die Zusammensetzung in Form eines Granulats für die orale Lösung vorliegt, **dadurch gekennzeichnet, dass** es die folgenden Schritte umfasst:
a) Granulieren des Ibuprofens mit einer Lösung von Tramadol oder eines seiner pharmazeutisch verträglichen Salze und einem Bindemittel
b) Trocknen der Granulate
c) Mischen der getrockneten Granulate mit Arginin

12. Verfahren gemäss Anspruch 10, wobei die Zusammensetzung in Form einer oralen Lösung vorliegt, **dadurch gekennzeichnet, dass** es die folgenden Schritte umfasst:
a) Lösen des Tramadols oder eines seiner pharmazeutisch verträglichen Salze in einer ausreichenden Menge Wasser
b) Anschliessende Zugabe der basischen Aminosäure bis zur vollständigen Dispersion
c) Anschliessende Zugabe des Ibuprofen bis zu seiner vollständigen Auflösung

13. Verfahren gemäss Anspruch 10, wobei die Zusammensetzung in Form einer Tablette vorliegt, **dadurch gekennzeichnet, dass** es die folgenden Schritte umfasst:
a) Mischen von Tramadol oder eines seiner pharmazeutisch verträglichen Salze mit Ibuprofen
b) Granulieren der in vorherigem Schritt erhaltenen Mischung mit einer Lösung eines Bindemittels
c) Trocknen der erhaltenen Granulate
d) Mischen der trockenen Granulate mit Arginin
e) Komprimierung

14. Verwendung einer Zusammensetzung nach einem der Ansprüche 1 bis 9 zur Herstellung eines Medikaments, das für die Behandlung von mindestens einem Leiden ausgewählt aus Schmerz, Entzündung und Fieber oder jeder anderen Krankheit, die mit Ibuprofen und / oder Tramadol gelindert werden kann, bestimmt ist.

15. Verwendung gemäss Anspruch 14, **dadurch gekennzeichnet, dass** besagte Schmerzen chronisch oder akut sind und von mittlerer bis schwerer Intensität.

16. Verwendung gemäss eines der Ansprüche 14 oder 15, **dadurch gekennzeichnet, dass** die Schmerzen ausgewählt sind aus der Gruppe bestehend aus Cephalea, Odontalgie, Ohrenschmerzen, Dysmenorrhoe, Migräne, Muskelschmerzen, Gelenkschmerzen, Osteoarthritis, neuropathische Schmerzen, posttraumatische Schmerzen, postoperative Schmerzen und onkologische Schmerzen.

## Revendications

1. Composition pharmaceutique par voie orale, **caractérisée en ce qu'**elle comprend du tramadol ou l'un de ses sels pharmaceutiques acceptables, de ibuprofène et de l'arginine amino-acide basique, la proportion molaire entre l'aminocide et l'ibuprofène se trouvant entre 0,9 :1 et 1.1 :1

2. Composition selon les revendications 1, **caractérisée en ce que** l'aminocide est la L-arginine.

3. Composition selon les revendications 1 ou 2, **caractérisée en ce que** l'ibuprofène est sélectionné parmi le groupe formé par (R,S)-ibuprofène et (S)-ibuprofène.

4. Composition selon la revendication 3, **caractérisée en ce qu'**elle comprend du tramadol sous forme de hydrochloride de tramadol.

5. Composition selon l'une quelconque des revendications précédentes **caractérisée en ce qu'**elle comprend une proportion molaire entre l'arginine et l'ibuprofène de 1 :1

6. Composition selon l'une quelconque des revendications précédentes sous forme de solide comprenant les quantités suivantes d'ibuprofène et de tramadol :
- entre 50 et 800 mg de (R,S)-ibuprofène ou une quantité thérapeutiquement équivalente de (S)-ibuprofène, et
- entre 20 et 300 mg de tramadol ou une quantité thérapeutiquement équivalente d'un de ses sels pharmaceutiquement acceptables.

7. Composition selon l'une quelconque des revendications précédentes sous forme liquide, comprenant les quantités suivantes d'ibuprofène et de tramadol :
- entre 20 mg / ml et 200 mg / ml de (R,S)-ibuprofène ou une quantité thérapeutiquement équivalente de (S)-ibuprofène, et
- entre 20 mg /ml et 200 mg / ml de tramadol ou une quantité thérapeutiquement équivalente d'un de ses sels pharmaceutiquement acceptables.

8. Composition selon l'une quelconque des revendications 5 et 6, comprenant ce qui suit en tant que le seul principe actif :
- 400 mg de (R,S) d'ibuprofène ou une quantité thérapeutiquement équivalente de (S)-ibuprofène,
- 340 mg d'arginine et
- 75 mg de hydrochloride de tramadol
a) en une dose unique, s'il s'agit d'une composition solide, ou
b) pour chaque 2 à 6 ml, s'il s'agit d'une composition liquide.

9. Composition selon l'une quelconque des revendications 1 à 8 dont la forme pharmaceutique est sélectionnée parmi le groupe formé par des granules pour solution orale, solutions orales ou tablettes.

10. Procédé pour préparer l'une quelconque des compositions des revendications 1 à 8, consistant à mélanger du tramadol ou l'un de ses sels pharmaceutiquement acceptables, de l'ibuprofène et de l'amino-acide basique mentionné, avec au moins un excipient pharmaceutiquement acceptable.

11. Procédé selon la revendication 10, la composition mentionnée étant sous forme de granules pour solution orale, **caractérisé en ce qu'**il comprend les phases suivantes :
a) granuler de l'ibuprofène avec une solution de tramadol ou l'un de ses sels pharmaceutiquement acceptable et avec un agent liant,
b) faire sécher les granules
c) mélanger les granules sèches avec de l'arginine

12. Procédé selon la revendication 10, la composition mentionnée étant sous forme d'une solution orale, **caractérisé en ce qu'**il comprend les phases suivantes :
a) dissoudre le tramadol ou l'un de ses sels pharmaceutiquement acceptables dans une quantité suffisante d'eau,
b) ajouter après l'amino-acide basique jusqu'à l'obtention d'une dispersion totale et
c) ajouter ensuite de l'ibuprofène jusqu'à l'obtention d'une dissolution totale.

13. Procédé selon la revendication 10, la composition mentionnée étant sous forme de tablettes, **caractérisé en ce qu'**il comprend les phases suivantes :
a) mélanger du tramadol ou l'un de ses sels pharmaceutiquement acceptable et de l'ibuprofène,
b) granuler le mélange obtenu dans la phase antérieure avec une solution d'un agent liant,
c) faire sécher les granules obtenues
d) mélanger les granules sèches avec de l'arginine
e) comprimer

14. Usage d'une composition selon l'une quelconque des revendications 1 à 9 pour la fabrication d'un médicament destiné au traitement d'au moins un malaise tel que douleur, inflammation et fièvre ou autre malaise pouvant être allégé avec de l'ibuprofène et / ou du tramadol.

15. Usage selon la revendication 14, **caractérisé en ce que** la douleur mentionnée est une douleur chronique ou aigüe, d'intensité modérée ou sévère.

16. Usage selon l'une quelconque des revendications 14 ou 15, **caractérisé en ce que** la douleur est comprise dans le groupe formé par céphalée, odontalgie, otalgie, dysménorrhée, migraine, douleur musculaire, douleur articulaire, ostéo-arthrite, douleur neuropathique, douleur post-traumatique, douleur post-chirurgicale et douleur oncologique.
